# EUROPEAN PATENT APPLICATION

(11) **EP 4 643 677 A1**
(43) Date of publication of application: **05.11.2025**
(21) Application number: 25166375.3
(22) Date of filing: 26.03.2025
(51) Int. Cl.: A24F 40/05, A24F 40/10, A61M 15/00

(54) **A VAPOR INHALATION DEVICE**

(30) Priority: 26.03.2024 MY PI2024001831
(71) Applicant: Cape EMS Manufacturing (M) SDN BHD, 81400 Senai Johor (MY)
(72) Inventor: BOON, Tan Juan, 81750 Masai Johor (MY); WU, Edmund, 80400 Johor Bahru Johor (MY)
(74) Representative: Papula Oy

(57) **Abstract**

The present invention discloses a vapor inhalation device comprising a power source (100); a reservoir (200) for holding liquid to be vaporized; an ultrasonic module (300) disposed downstream, in terms of fluid flow, of the reservoir (200) for vaporizing the liquid; a frequency adjustment module (400) operatively connected to the ultrasonic module (300) for adjusting a frequency of vibration of said ultrasonic module (300); and a delivery system (500) for directing flow of vapor through a mouthpiece (501) to a user; wherein the ultrasonic module (300) is configured to vibrate at variable frequencies to atomize liquid drawn from the reservoir (200), causing the vapor to be formed of different densities based on the frequency of vibration and exit through the delivery system (500).

## Description

### FIELD OF INVENTION

The present invention relates to a vapor inhalation device. More particularly, the present invention relates to a vapor inhalation device utilizing a non-heating mechanism to vaporize fluid.

### BACKGROUND OF THE INVENTION

Electronic cigarettes have significantly evolved from their initial designs, which predominantly used heating elements like coils to vaporize e-liquid. Traditionally, these coils generated heat to convert the liquid into inhalable vapor. However, a substantial shift has occurred in recent years, leading to the emergence of e-cigarettes employing non-heating elements. Modern vaping devices, such as pod systems and heat-not-burn mechanisms, have introduced innovations including ultrasonic technology or induction to create vapor without relying on direct heat application. This evolution addresses several issues associated with coil-based systems, including leakage, overheating, and potential byproduct risks. The transition to non-heating elements offers notable advantages, including enhanced safety measures, reduced maintenance requirements, and a more controlled vaporization process. Moreover, these innovations aim to provide users with a smoother and more reliable vaping experience while potentially minimizing health concerns associated with traditional coil-based e-cigarettes. This shift signifies a pivotal step toward the development of more efficient and safer vaping technologies, aligning with the industry's pursuit of enhanced user satisfaction and reduced health risks.

Many technologies have been implemented to improve on devices for delivering vapor. One such example is a United States patent with publication no. US20160213866A1 which discloses an apparatus for ultrasonic atomizing of a liquid in a container, comprising a housing with a power source, the housing having a mouthpiece and the container having the liquid for atomizing, the container has a wick for drawing the liquid to a probe on an ultrasonic transducer, which is located in the housing, the probe engages the wick and allows for a displacement of greater than 10 micrometer, the ultrasonic transducer is connected to the power source and a signal generator, whereby when activated, the ultrasonic transducer vibrates the probe, such that the liquid from the wick is atomized, and vapor of the atomized liquid exits the mouthpiece.

Another technology as disclosed in an international publication WO2017175218A2 recites a mobile inhaler comprising a mouthpiece comprising at least one inhaling opening, a body element connected to the mouthpiece and comprising a housing for holding a DC power source, a power conversion unit comprising an inverter operative to convert a DC voltage provided by a DC power source, into a higher AC voltage, a nebulizer comprising an ultrasonic vibrator and a mesh, wherein said ultrasonic vibrator is activated by said AC voltage, and wherein said nebulizer is adapted to enable converting into aerosol droplets at least part of a liquid comprised in an enclosure, and the aerosol droplets to be directly inhaled from said mouthpiece, and an enclosure adapted to enable holding a liquid, and characterized in that it provides a connection to enable fluid transfer from said enclosure to the nebulizer.

A United States patent with publication no. US20210345664A1 discloses an ultrasonic atomization sheet, comprising a flaky piezoelectric substrate, a surface electrode attached to one surface of the piezoelectric substrate, and a drive electrode attached to the other surface of the piezoelectric substrate, wherein the piezoelectric substrate is elongated.

Yet another United States patent with publication no. US11641876B2 discloses an inclined ultrasonic atomizing sheet structure, an atomizer, and an electronic cigarette.

The inclined ultrasonic structure comprises atomization cotton and an ultrasonic atomizing sheet. The ultrasonic atomizing sheet is obliquely arranged relative to the horizontal plane, and two ends of the atomization cotton are used for connecting to tobacco tar in a tobacco tar cavity via tobacco tar guide cotton, or the two ends of the atomization cotton are directly arranged in the tobacco tar cavity. A lower surface of the atomization cotton is in contact with an atomization surface of the ultrasonic atomizing sheet. The ultrasonic atomizing sheet structure can prevent the ultrasonic atomizing sheet from being soaked in the tobacco tar and thus prevent the situation that smoke cannot be produced by atomization.

### SUMMARY OF INVENTION

An object of the present invention is to provide an alternative method of generating vapor by means of ultrasonic vibration. It is also an object of the present invention to enhance the vaping experience of the user through the honeycomb configuration of the delivery channel of the device.

In one aspect of the present invention, there is provided a vapor inhalation device comprising a power source; a reservoir for holding liquid to be vaporized; an ultrasonic module disposed downstream, in terms of fluid flow, of the reservoir for vaporizing the liquid; a frequency adjustment module operatively connected to the ultrasonic module for adjusting a frequency of vibration of said ultrasonic module; and a delivery system for directing flow of vapor through a mouthpiece to a user; wherein the ultrasonic module is configured to vibrate at variable frequencies to atomize liquid drawn from the reservoir, causing the vapor to be formed of different densities based on the frequency of vibration and exit through the delivery system.

Preferably, the device further comprising a liquid channel which is provided for drawing liquid from the reservoir for atomization.

Preferably, the liquid channel is provided with a unidirectional liquid pump for pushing the liquid from the reservoir into a tank segment disposed above said reservoir.

Preferably, the tank segment is configured to house the ultrasonic module and ensure said ultrasonic module is constantly submerged in the liquid drawn from the reservoir.

Preferably, the tank segment is equipped with a sensor that triggers the unidirectional liquid pump to push more liquid into said tank segment when the ultrasonic module is not fully submerged in the liquid.

Preferably, the device further comprising a unidirectional air inlet is provided for an ingress of air downstream, in terms of fluid flow, of the ultrasonic module.

Preferably, the device further comprising a vapor-permeable membrane fitted at a top portion of the tank segment, which is configured to prevent liquid from entering the delivery system and only allowing vapor produced through atomization of the liquid to pass through said membrane.

Preferably, the ultrasonic module is activated by a draw action of inhaling through the mouthpiece of the delivery system.

Preferably, the ultrasonic module comprises an actuator, a micro induction coil, or a combination thereof.

Preferably, the actuator is a microelectromechanical system cantilever or diaphragm.

Preferably, the delivery system is formed with a channel configured with a plurality of baffles arranged in a manner to enhance the acceleration of the vapor through the channel when said vapor is inhaled or released to the user.

Preferably, the baffles comprise a plurality of fins arranged in a manner that forms a honeycomb arrangement.

Preferably, the delivery system is further formed with a unidirectional air inlet for the ingress of air downstream, in terms of fluid flow, of the baffles.

In another aspect of the present invention, there is provided a vapor inhalation device having a delivery system comprising an ultrasonic module for vaporizing liquid; and a delivery system for directing flow of vapor through a mouthpiece to a user, being formed with a channel configured with a plurality of baffles arranged in a manner that enhances the acceleration of the vapor through the channel of the delivery system when said vapor is inhaled or released to the user through the mouthpiece; wherein the ultrasonic module is configured to vibrate at variable frequencies to atomize the liquid, causing the vapor to be formed of different densities based on the frequency of vibration and exit through the delivery system.

Preferably, the baffles comprise a plurality of fins arranged in a manner that forms a honeycomb arrangement.

Preferably, the delivery system is further formed with a unidirectional air inlet for the ingress of air downstream, in terms of fluid flow, of the baffles.

One skilled in the art will readily appreciate that the present invention is well adapted to carry out the objects and obtain the ends and advantages mentioned, as well as those inherent therein. The embodiment described herein is not intended as limitations on the scope of the invention.

### BRIEF DESCRIPTION OF THE DRAWINGS

For the purpose of facilitating an understanding of the invention, there is illustrated in the accompanying drawing the preferred embodiments from an inspection of which when considered in connection with the following description, the invention, its construction and operation and many of its advantages would be readily understood and appreciated.
**FIG. 1** illustrates a preferred embodiment of a vapor inhalation device.
**FIG. 2** illustrates an alternative embodiment of the vapor inhalation device.
**FIG. 3** illustrates an exemplary embodiment of an ultrasonic module of the device.
**FIG. 4** illustrates another exemplary embodiment of the ultrasonic module of the device.
**FIG. 5** illustrates an exemplary embodiment of a delivery system of the vapor inhalation device.
**FIG. 6** illustrates another exemplary embodiment of the delivery system of the vapor inhalation device.

### DETAILED DESCRIPTION OF THE INVENTION

Hereinafter, the invention shall be described according to the preferred embodiments of the present invention and by referring to the accompanying description and drawings. However, it is to be understood that limiting the description to the preferred embodiments of the invention is merely to facilitate discussion of the present invention and it is envisioned that those skilled in the art may devise various modifications without departing from the scope of the appended claim.

The invention will now be described in greater detail, by way of example, with reference to the drawings.

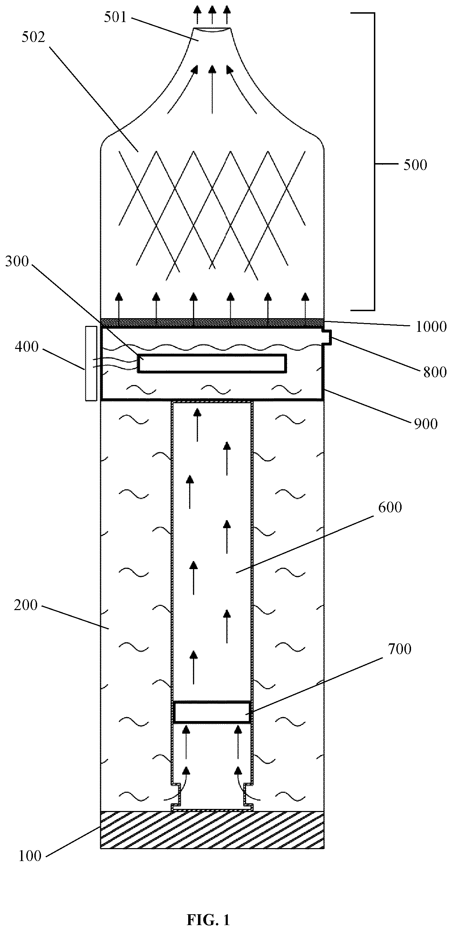

**FIG. 1** illustrates a preferred embodiment of a vapor inhalation device according to the present invention. The device comprises an elongated housing as exemplified herein may be substantially cylindrical in shape and may have a hollow interior comprising one or more segments, such that it is able to contain a plurality of components including, but is not limited to, a power source **100**, a reservoir **200** for holding liquid to be vaporized, an ultrasonic module **300** disposed downstream, in terms of fluid flow, of the reservoir **200** for vaporizing the liquid, a frequency adjustment module **400** operatively connected to the ultrasonic module **300**, and a delivery system **500** for directing flow of vapor through a mouthpiece **501** to a user. Preferably, the elongated housing may be made from a variety of materials including metals, metal alloys, plastic such as polycarbonate, rubberized coatings, or the likes. Additionally, the elongated housing may also be configured with a unibody design, such that the delivery system **500** may be non-detachable, in which the delivery system **500** may be press fitted and sealed to prevent any leakage or entry into the device. Optionally, the delivery system **500** may be detachable from the elongated housing via a magnetic mechanism, typically used within electronic cigarettes for easy maintenance and replacement of the delivery system. Additionally, the mouthpiece **501** may be a part of the delivery system **500**, whereby said mouthpiece **501** is manufactured as one piece with said delivery system **500**, but may also be separable from said delivery system **500**, such that different types of mouthpieces **501** may be utilized.

In this embodiment, the power source **100** may be disposed at a bottom portion within the elongated housing, at a position below the reservoir **200**, in which the power source **100** may be a type of built-in battery which may be integrated into the device, such as lithium-ion (Li-ion) batteries. Li-ion batteries are a type of rechargeable batteries that are lightweight and compact, which is suitable for this type of device. Preferably, the power source **100** may also be encapsulated within the device and placed in a dedicated compartment, such that the enclosure may be designed to prevent the liquid or other contaminants from reaching the power source **100**, ensuring the safety and proper function of said power source **100**. Besides that, the power source **100** may be integrated with a circuit board which not only functions to actuate the ultrasonic module **300** and receive signals from the frequency adjustment module **400**, but also manage safety factors for the power source **100** or the device, such as overcharging, over-discharging, and short circuits.

In this embodiment, the reservoir **200**, as mentioned above, may be disposed above the power source **100**, such that the reservoir **200** functions as a storage or container for liquid to be vaporized. By way of example, the liquid in this embodiment may be an e-liquid for use is electronic cigarettes or some form of medication in liquid or powder form that can be aerosolized and inhaled by the users. Preferably, the liquid stored in the reservoir **200** may be of a particular viscosity range for the device to effectively vaporize or atomize said liquid. For example, the viscosity of medications used in devices such as nebulizers typically falls within a range that is lower than e-liquids used in electronic cigarettes or vaping devices. Medications are often formulated as solutions or suspensions with viscosities ranging from substantially 1 to 15 centipoises (cP), although this may vary depending on the specific medication and its concentration. In contrast, e-liquids used for vaping are formulated with slightly higher viscosities, with viscosities ranging from around 50 to 100 centipoises (cP) or higher, depending on factors such as a ratio of propylene glycol (PG) to vegetable glycerin (VG) in its formulation, and a presence of additional flavorings or additives. Additionally, the reservoir **200** may also be detachable from the elongated housing, such that said reservoir **200** may be refillable with the liquid as said reservoir **200** becomes empty.

This configuration reduces wastage, particularly plastic wastage, as the device would not need to be disposed of after full usage.

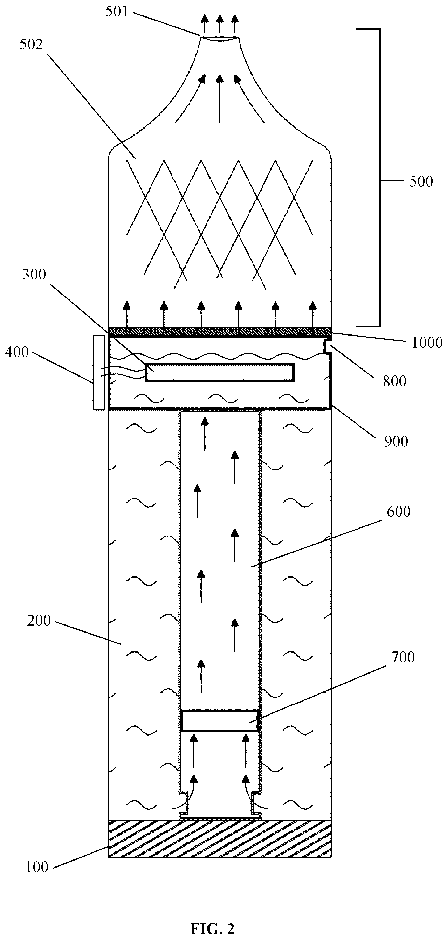

Referring to **FIG. 1** and **FIG. 2**, disposed within the reservoir **200** is a liquid channel **600** which is provided for drawing liquid from said reservoir **200** to a tank segment **900** that is disposed at a position above the reservoir **200**. The liquid channel **600** may be a cylindrical tube or capillary tube with inlets at a size large enough to allow the liquid to be drawn into it by mere pressure. However, to counter the effects of gravity on the liquid drawn from the reservoir **200** into the liquid channel 600, said liquid channel 600 may be provided with a unidirectional liquid pump **700** for pushing the liquid downstream, in terms of fluid flow, to the tank segment **900**. Preferably, the tank segment **900** may be configured to house the ultrasonic module **300** and ensures that the liquid drawn from the reservoir **200** into the tank segment **900** fully submerges said ultrasonic module **300**.

To ensure that the ultrasonic module **300** is constantly fully submerged in the tank segment **900**, said tank segment **900** may be equipped with a sensor that may trigger the unidirectional liquid pump **700** to push more liquid into said tank segment **900**, when liquid level drops below a predetermined level below the ultrasonic module **300**. As a result, the liquid may then fill the tank segment **900** to a level that an air pocket may be formed above the ultrasonic module **300**, wherein the air pocket allows for vapor to be collected within the tank segment **900** upon atomization by said ultrasonic module **300**. Once the level has been reached, the sensor may then trigger the unidirectional liquid pump **700** to stop drawing liquid any further. Further, both the sensor and the unidirectional liquid pump **700** may be directly connected to the power source **100** and may be configured to be on a standby mode, in a way that conserves energy to prevent energy of said power source **100** from being constantly drained.

Besides that, the tank segment **900** may also be fitted with a vapor-permeable membrane **1000** at its top portion which may be configured to prevent liquid from entering the delivery system **500** and only allowing vapor produced through atomization of the liquid to pass through said membrane **1000**. By way of example, the vapor-permeable membrane **1000** is a semi-permeable membrane that may be made from a variety of materials, both rigid or flexible, such as polyethylene (PE), polypropylene (PP), polyurethane (PU), microporous films, porous ceramic, or the likes. Further, materials such as PE may be treated to enhance their vapor permeability while maintaining their liquid barrier properties.

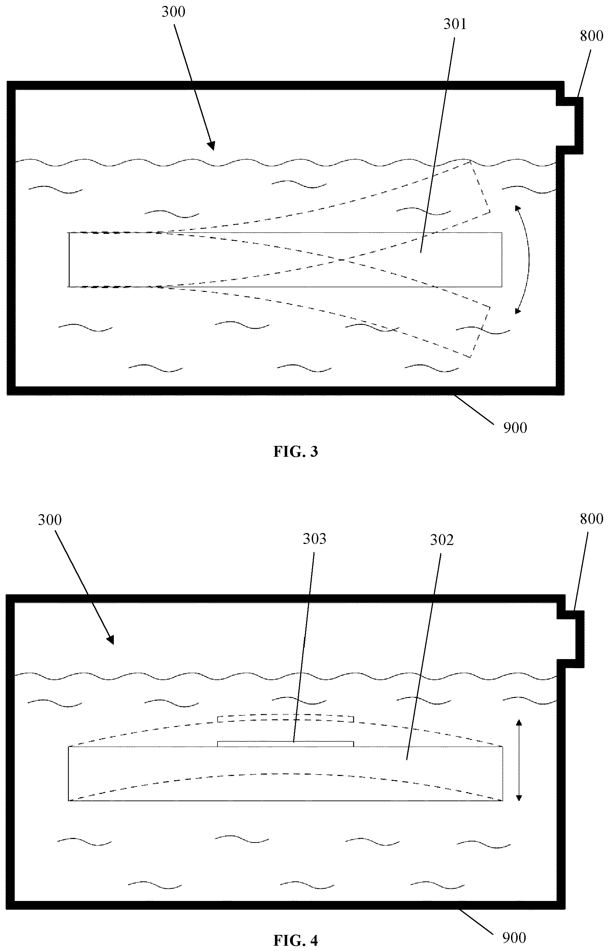

With reference to **FIG. 3** and **FIG. 4**, the ultrasonic module **300** comprises an actuator **301, 302** or a micro induction coil **303** attached to said actuator **301, 302** configured to vibrate at variable frequencies to atomize liquid drawn from the reservoir **200**, causing the vapor to be formed of different densities based on the frequency of the vibration and exit through the delivery system **500**. Preferably, the actuator **301, 302** may be a microelectromechanical system (MEMS) cantilever **301** or a MEMS diaphragm **302**, with said diaphragm **302** being coupled with the induction coil **303**. The MEMS cantilever **301** may be a thin beam anchored at one end and is free to move or deflect at an opposite end, in which an amount of deflection or bend caused by an external force, such as pressure, acceleration, or mass, is applied to the free end of the cantilever. On the other hand, the MEMS diaphragm **302** is a thin, flexible membrane that can deform in response to external pressure or force, such that said MEMS diaphragm **302** either deflects or deforms and this deformation may be detected by means of various transduction methods, such as capacitance changes, piezoresistive effects, or optical interferometry. The induction coil **303** which is coupled with the MEMS diaphragm **302** may be used to detect vibration caused by events such as airflow changes or liquid movement within the device.

In this embodiment, the device may be activated by a draw action of inhaling through the mouthpiece **501** of the delivery system **500**, in which air can be drawn into the device to facilitate the movement of vapor to the delivery system **500** from a unidirectional air inlet **800** disposed at either side of the tank segment 900, such that said air inlet **800** may be provided for an ingress of air downstream, in terms of fluid flow, of the ultrasonic module **300**. Preferably, the intake of air may be detected by the induction coil **303** as mentioned above, which then triggers the power source **100** to actuate the ultrasonic module **300**, subsequently initiating the vibration of the MEMS cantilever **301** or MEMS **302** diaphragm and vaporize or atomize the liquid to form vapor. Also preferably, the air inlet **800** may be configured in a culvert-type structure, in which said air inlet **800** may be slightly extending out from the tank segment **900**. Besides that, the air inlet **800** may also be parallel to the air pocket in the tank segment **900** to facilitate the flow of vapor downstream, in terms of fluid flow, to the delivery system **500.** **FIG. 2** illustrates an alternative configuration of the air inlet **800**, whereby instead of extending outwards from the tank segment **900**, the air inlet **800** may extend downwards into said tank segment **900**. These configurations prevent leaking of the liquid within the reservoir **200** out from the device if the device is tilted or placed on its side.

In a preferred embodiment, the ultrasonic module **300** may be operatively connected to the frequency adjustment module **400**, such that said frequency adjustment module **400** may be configured for adjusting a frequency of vibration of the ultrasonic module **300**, wherein the variable frequencies as adjusted causes vapor to form having different densities based on said frequencies. Typically, an optimum frequency range to vaporize liquid depends on various factors such as type of liquid, intended application, and specific vaporization being used. In general, the frequency range for vaporizing liquids may fall within the ultrasonic frequency range between 20 kHz to several megahertz. For medical applications where drug delivery is applied using an ultrasonic nebulizer, the frequency range of operation may range between substantially 1 MHz to substantially 3 MHz, such that fine aerosols or mists with droplet sizes suitable for inhalation into the respiratory system may be formed. Similarly for e-liquid used in electronic cigarettes, the optimum frequency range may be between 1 MHz to 2 MHz.

By way of example, the frequency adjustment module **400** may be configured in the form of a slide switch that uses a slider mechanism to adjust the frequency of vibration, or a button that is clickable to toggle between a plurality of predetermined frequencies. Preferably, the frequency adjustment module **400** may be positioned at an opposite end of the tank segment **900** relative to the unidirectional air inlet **800.** Optionally, the frequency adjustment module **400** may also be operatively connected to the integrated chip in the power source **100**, which then adjusts the frequency being output by the ultrasonic module **300** accordingly. Advantageously, the ultrasonic module **300** in comparison to conventional coils used in vaporizing liquid, operates at a substantially low temperature that not only helps maintain a chemical stability of vapor produced, but also reduces or potentially eliminates emission of any potential toxins. Further, the no-coil design further eliminates dry hits in the absence of liquid drawn from the reservoir **200** through the liquid channel **600**.

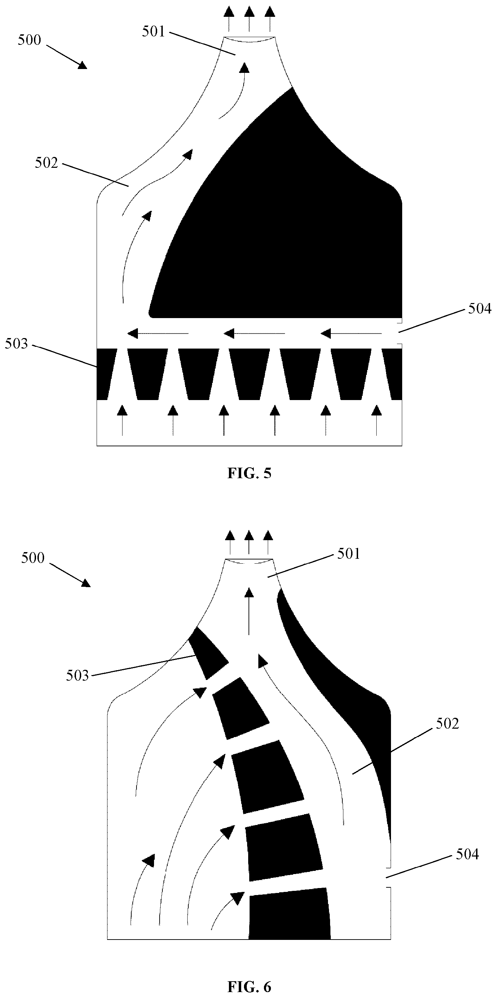

Referring to **FIG. 5** and **FIG. 6**, the delivery system **500** may be configured in a way that includes a channel **502** that is configured with a plurality of baffles **503** that are arranged in a manner that enhances the acceleration of the vapor through the channel **502** when said vapor is inhaled or released to the user through the mouthpiece **501**. The baffles **503** are structures or barriers placed within the channel **502** of the delivery system **500** to control the flow of the vapor being inhaled or released to the user. Preferably, the baffles **503** may comprise a plurality of fins which are arranged in a manner that forms a honeycomb arrangement, either on a horizontal plane or on a vertical plane, such as illustrated in **FIG. 5** and **FIG. 6** respectively. The arrows as illustrated depict the flow of vapor through the channel **502** in the delivery system **500**. Further, the delivery system **500** may further include another unidirectional air inlet **504** disposed on either side portion of the delivery system **500**, which may be configured for facilitating the flow of vapor produced from the ultrasonic module **300.** In a preferred embodiment, the configuration of the baffles **503** with the addition of the unidirectional air inlet **504** purposefully induces a Venturi effect, which is a particular application of Bernoulli's principle. Bernoulli's principle states that in a flowing fluid, an increase in the speed of the fluid occurs simultaneously with a decrease in pressure or a decrease in the fluid's potential energy, whereas the Venturi effect denotes a reduction in fluid pressure that occurs when a fluid flows through a constricted section of a pipe or channel, followed by an expansion in the pipe's cross-sectional area. In this context, as according to **FIG. 5** and **FIG. 6**, the baffles **503** as arranged in the honeycomb arrangement creates smaller passages for vapor to flow through, which as according to the Venturi effect, increases a velocity of the vapor flow when said vapor is being inhaled or released to the user. Advantageously, this configuration enhances the vapor delivery to the user as the user requires less effort in inhaling through the device to obtain a desired amount of vapor.

The present disclosure includes as contained in the appended claims, as well as that of the foregoing description. Although this invention has been described in its preferred form with a degree of particularly, it is understood that the present disclosure of the preferred form has been made only by way of example and that numerous changes in the details of construction and the combination and arrangements of parts may be resorted to without departing from the scope of the invention.

## Claims

**1.** A vapor inhalation device comprising
a power source (100);
a reservoir (200) for holding liquid to be vaporized;
an ultrasonic module (300) disposed downstream, in terms of fluid flow, of the reservoir (200) for vaporizing the liquid;
a frequency adjustment module (400) operatively connected to the ultrasonic module (300) for adjusting a frequency of vibration of said ultrasonic module (300); and
a delivery system (500) for directing flow of vapor through a mouthpiece (501) to a user;
wherein the ultrasonic module (300) is configured to vibrate at variable frequencies to atomize liquid drawn from the reservoir (200), causing the vapor to be formed of different densities based on the frequency of vibration and exit through the delivery system (500).

**2.** The device as according to claim 1, further comprising a liquid channel (600) which is provided for drawing liquid from the reservoir (200) for atomization.

**3.** The device as according to claim 2, wherein the liquid channel (600) is provided with a unidirectional liquid pump (700) for pushing the liquid from the reservoir (200) into a tank segment disposed above said reservoir (200).

**4.** The device as according to claim 3, wherein the tank segment (900) is configured to house the ultrasonic module (300) and ensure said ultrasonic module (300) is constantly submerged in the liquid drawn from the reservoir (200).

**5.** The device as according to claims 3 or 4, wherein the tank segment (900) is equipped with a sensor that triggers the unidirectional liquid pump (800) to push more liquid into said tank segment (900) when the ultrasonic module (300) is not fully submerged in liquid.

**6.** The device as according to any one of the preceding claims, further comprising a unidirectional air inlet (800) is provided for an ingress of air downstream, in terms of fluid flow, of the ultrasonic module (300).

**7.** The device as according to any one of the preceding claims, further comprising a vapor-permeable membrane (1000) fitted at a top portion of the tank segment (900), which is configured to prevent liquid from entering the delivery system (500) and only allowing vapor produced through atomization of the liquid to pass through said membrane (1000).

**7.** The device as according to any one of the preceding claims, wherein the ultrasonic module (300) is activated by a draw action of inhaling through the mouthpiece (501) of the delivery system (500).

**8.** The device is according to any one of the preceding claims, wherein the ultrasonic module (300) comprises an actuator (301, 302), a micro induction coil, or a combination thereof.

**9.** The device as according to claim 8, wherein the actuator (301, 302) is a microelectromechanical system cantilever (301) or diaphragm (302).

**10.** The device as according to claim any one of the preceding claims, wherein the delivery system is formed with a channel (502) configured with a plurality of baffles (503) arranged in a manner to enhance the acceleration of the vapor through the channel (502) when said vapor is inhaled or released to the user.

**11.** The device as according to claim 10, wherein the baffles (503) comprise a plurality of fins arranged in a manner that forms a honeycomb arrangement.

**12.** The device according to claims 10 or 11, wherein the delivery system (500) is further formed with a unidirectional air inlet (504) for the ingress of air downstream, in terms of fluid flow, of the baffles (503).

**13.** A vapor inhalation device having a delivery system (500) comprising
an ultrasonic module (300) for vaporizing liquid; and
a delivery system (500) for directing flow of vapor through a mouthpiece (501) to a user, being formed with a channel (502) configured with a plurality of baffles (503) arranged in a manner that enhances the acceleration of the vapor through the channel (502) of the delivery system (500) when said vapor is inhaled or released to the user through the mouthpiece (501);
wherein the ultrasonic module (300) is configured to vibrate at variable frequencies to atomize the liquid, causing the vapor to be formed of different densities based on the frequency of vibration and exit through the delivery system (500).

**14.** The device according to claim 13, wherein the baffles (503) comprise a plurality of fins arranged in a manner that forms a honeycomb arrangement.

**15.** The device according to claims 13 or 14, wherein the delivery system (500) is further formed with a unidirectional air inlet (504) for the ingress of air downstream, in terms of fluid flow, of the baffles (503).
